# EUROPEAN PATENT SPECIFICATION

(11) **EP 3 713 621 B1**
(45) Date of publication and mention of the grant of the patent: **07.06.2023**
(21) Application number: 18920049.6
(22) Date of filing: 27.11.2018
(51) Int. Cl.: A61M 1/00, A61M 5/178, C12M 1/00

(54) **A TIP SEAL ARRANGEMENT FOR INJECTORS CONTAINING STEM CELLS**
SPITZENDICHTUNGSANORDNUNG FÜR INJEKTOREN MIT STAMMZELLEN
AGENCEMENT DE JOINT DE POINTE POUR INJECTEURS CONTENANT DES CELLULES SOUCHES

(30) Priority: 28.11.2017 TR 201718999
(43) Date of publication of application: 30.09.2020
(73) Proprietor: Tiryaki, Kemal Tunc, Istanbul 34077 (TR)
(72) Inventor: Tiryaki, Kemal Tunc, Istanbul 34077 (TR)
(74) Representative: Gülser Kinali, Zeynep
(86) International application number: PCT/TR2018/050732
(87) International publication number: WO 2019/226127

(56) References cited:
- WO-A1-85/00524
- WO-A2-2004/104553
- JP-U- 3 213 458
- JP-U- 3 213 458
- US-A1- 2003 040 701
- US-A1- 2006 224 144
- US-A1- 2007 106 208

## Description

### Technical Field

The invention relates to an injector arrangement which allows stem cells obtained from tissues of a living organism to be transferred from their initial location to a centrifuge medium without being exposed to air or without experiencing any volume losses.

### Background Art

Stem cells are obtained from embryos, fetuses or tissues of the human body such as bone marrow, blood, fat etc. Stem cells that are available in the human body are progenitor cells which only possess the ability to transform into the cells of certain tissues, in contrast to embryonic stem cells.

Today, after having discovered the effectiveness of stem cells obtained from fat tissue especially in treatment of tissue losses and regeneration, various methods have been developed for obtaining stem cells from fat tissue. Accordingly, the fat tissue located at the lower abdomen or the inner thigh area is removed using one of the liposuction techniques known in the state of the art and transferred to the laboratory. After obtaining stem cells from the fat tissue sample, the said stem cells are put through several centrifuge process before transplantation. However, the centrifuge process is carried out using a regular centrifuge tube and volume losses are experienced during the transfer of the obtained stem cells from their initial tube, injector etc. to the centrifuge tube. The aforementioned reasons, developing an injector arrangement that prevents volume losses during the centrifuge process is required.

An application in the prior art is JP3213458U which relates to a multipurpose specimen collector having a function of collecting, preserving, centrifuging, and the like leakage of a specimen such as blood, and effectively replacing a test tube. After the specimen is collected in the outer tube, the needle and the plunger are removed, the outer casing is sealed with the first cap and the second cap, and the subsequent specimen centrifugal separation operation and the like are performed directly. Application numbered JP3213458U can not prevent leak of stem cells between the seal and the injector tube during centrifugation.

Therefore, the aforementioned disadvantages and lack of an adequate solution to the problem have made it necessary to develop an injector arrangement which allows stem cells obtained from tissues of a human body to be transferred from their initial location to a centrifuge medium without being exposed to air or without experiencing volume losses.

### Object of the Invention

The present invention relates to develop an injector arrangement which overcomes all disadvantages and provides further advantages, which allows stem cells obtained from tissues of a human body to be transferred from their initial location to a centrifuge medium without being exposed to air or without experiencing volume losses, due to the lack of an adequate solution to the problem in the prior art.

The primary object of the invention is to minimize the stem cell losses to be obtained during the centrifuge process of the split fat tissue, with the concave structure of the injector seal.

The object of the invention is to develop an injector arrangement, especially intended to be used to obtain fat tissue based stem cells, which allows stem cells obtained from the fat tissue from any preferred area of the individual's body to be delivered to the centrifuge process without requiring them to be transferred into a separate tube or injector. When the injector are centrifuged upside down, heavier stem cells are aggregated at the bottom of the concave arrangement and therefore the stem cell losses are minimized.

Another object of the invention is to develop an injector arrangement, especially intended to be used to obtain fat tissue based stem cells, which transfers the cells to be obtained to the centrifuge medium without exposing them to air.

Another object of the invention is to develop an injector arrangement, especially intended to be used to obtain fat tissue based stem cells, which transfers the cells to be obtained to the centrifuge medium without losing their vitality.

Another object of the invention is to develop an injector arrangement, especially intended to be used to obtain fat tissue based stem cells, which does not possess the risk of infection. Another object of the invention is to develop an injector arrangement, especially intended to be used to obtain fat tissue based stem cells, which transfers the stem cells to the centrifuge medium without experiencing volume losses.

Another object of the invention is to develop an injector arrangement, intended to be used to obtain fat tissue based stem cells, which eliminates risks such as infection and contamination.

Another object of the invention is to simplify the process by allowing the stem cells to be stored inside the injector into which they are initially drawn.

The present invention relates to an injector arrangement intended to be used to transfer the stem cells obtained from tissues of a living organism from their initial medium to the centrifuge medium without exposing them to air or without experiencing volume losses. Accordingly, the injector arrangement develop by the invention comprises a modular piston with a movable structure which allows drawing the fat tissue into the tube through a suction tip by means of a piston tip seal, a piston shaft with modular structure which allows the said modular piston to move inside the tube in forward and backward directions and which can be separated from the modular piston in order to allow the tube to be placed inside the present centrifuge device after the fat tissue is drawn inside the tube, and an O-ring which engages into the O-ring seat formed on the hollow locking part with independent structure in order to make the hollow locking part with an independent structure introduced on the said modular piston to act as a locking device during the centrifuge, or in other words, to prevent the fat tissue and split stem cells to leak outside the tube during the centrifuge, in order to allow the fat tissue to be drawn through a suction tip, stored inside a tube without intervention or exposure to air and centrifuged finally in order to obtain fat based stem cells. The injector arrangement developed by the present invention further comprises a piston tip seal with a concave structure introduced at the tip of the modular piston with movable structure.

In order to achieve the objects of the invention, a piston tip seal having a concave structure is introduced at the tip of the modular piston with movable structure mentioned in the invention. The concavity of the mentioned seal prevents the stem cells to leak in between the seal and the outer wall of the injector during the centrifuge process in order to prevent volume losses.

The structural and characteristic features and all of the advantages of the invention shall become apparent with the appended drawing and the detailed description with reference to those drawings. Therefore, assessment should be based on these drawing and the detailed description.

### Brief Description of the Drawings

- Figure-1.: A general perspective view of the injector arrangement of the invention.
- Figure-2.: An exploded perspective view of the injector arrangement of the invention.
- Figure-3.: An exploded perspective view of the injector arrangement of the invention from a different point of view.
- Figure-3.1: Exploded and assembled detail views of the modular piston heads of the invention showing close-ups of the O-ring and the O-ring seat.
- Figure-4.: A view of the piston shaft while disengaged from the threaded part.
- Figure-5.: A general view of an embodiment where concave injector seals are used.
- Figure-6,7,8.: Views of an injector embodiment where concave injector seals are used from different points of view.
- Figure-9, 10.: Views of the concave injector seal from different points of view.

The drawings should scaled and details not necessary to understand the invention may have been omitted. In addition, at least partially identical elements or elements with at least identical functions are indicated with same numbers.

### Parts References

- 100.: Injector arrangement
- 101.: Tube
- 102.: Piston tip seal
- 1021.: Positioning gap
- 103.: Modular Piston
- 1031.: Hollow Locking Part
- 1032.: Modular Piston Screw Hole
- 1033.: O-ring seat
- 104.: O-ring
- 105.: Piston shaft
- 106.: Threaded tip
- 107.: Piston handle
- 108.: Suction tip
- 109.: Piston shaft seat

### Detailed Description of the Invention

The present invention shall become apparent with the preferred embodiments of an injector arrangement, which allows stem cells obtained from fat tissue of a living organism, to be transferred from their initial location to a centrifuge medium without being exposed to air or without experiencing volume losses, given in the herein detailed description, without limiting the scope of the invention.

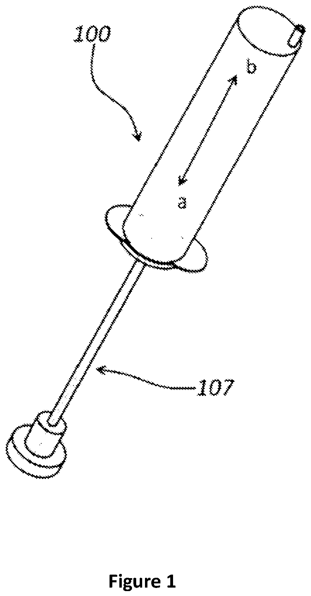

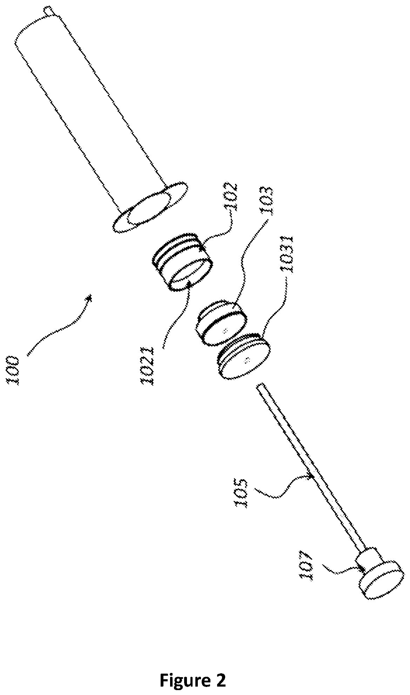

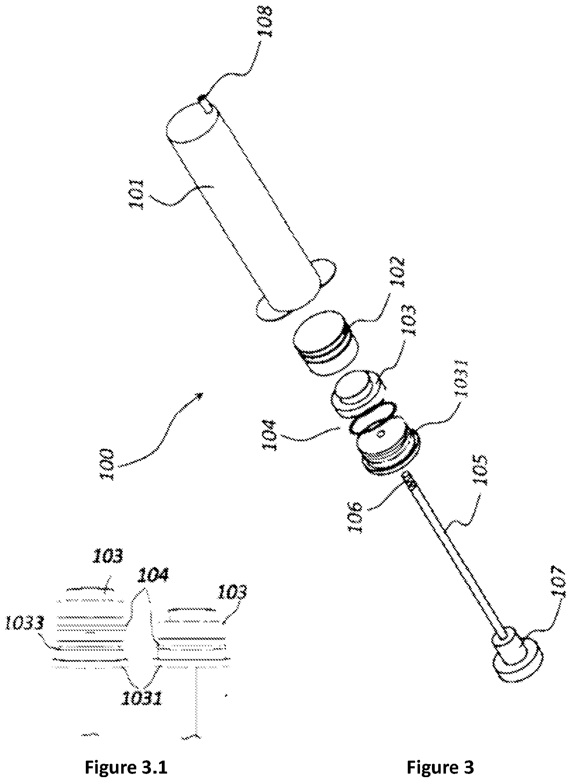

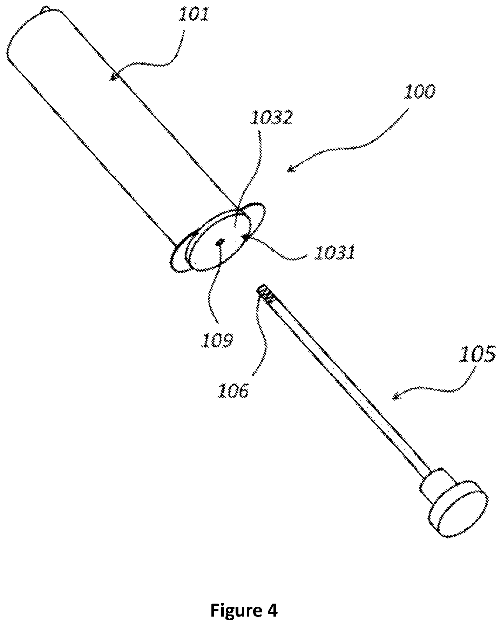

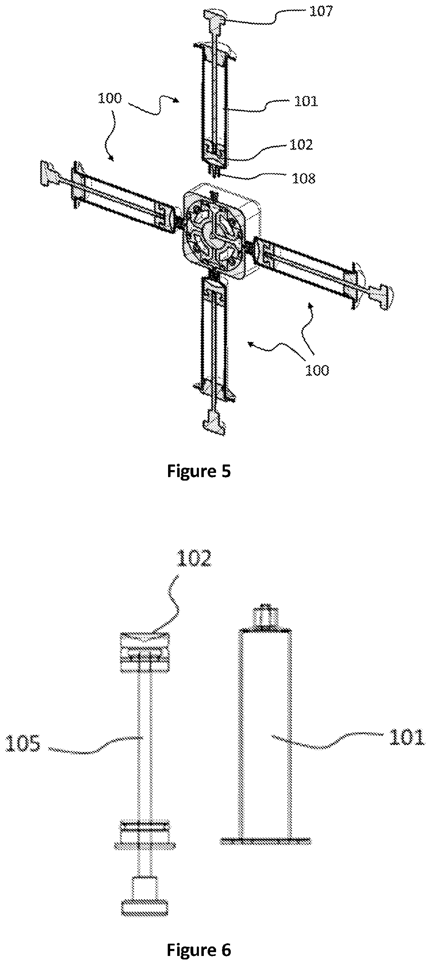

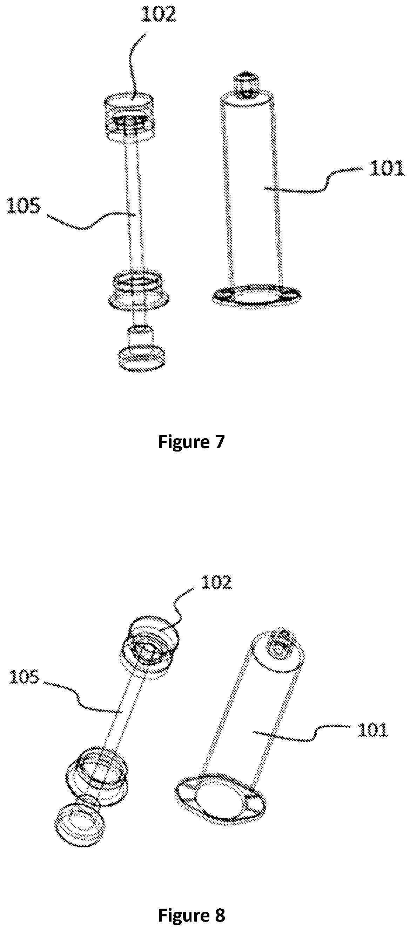

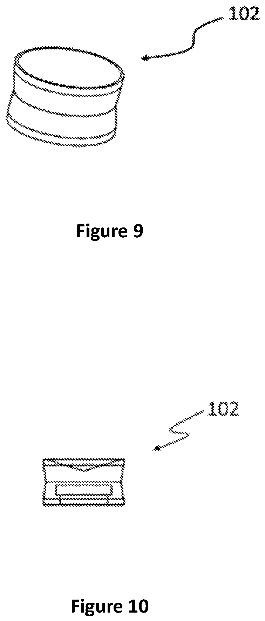

The present invention relates to an injector arrangement, whose example views are given in figures 1-5, which allows stem cells obtained from tissues of a human body to be transferred from their initial location to a centrifuge medium without being exposed to air or without experiencing volume losses. Accordingly, the injector arrangement comprises a modular piston (103) with a movable structure which allows drawing the fat tissue into the tube (101) through a suction tip (108) by means of a piston tip seal (102), a piston shaft (105) with modular structure which allows the said modular piston (103) to move inside the tube (101) in forward and backward directions and which can be separated from the modular piston (103) in order to allow the tube (101) to be placed inside the present centrifuge device after the fat tissue is drawn inside the tube (101), and an O-ring (104) which engages into the O-ring seat (1033) formed on the hollow locking part (1031) with independent structure in order to make the hollow locking part (1031) with an independent structure introduced on the said modular piston (103) to act as a locking device during the centrifuge, or in other words, to prevent the fat tissue and split stem cells to leak outside the tube (101) during the centrifuge. The injector arrangement developed by the present invention further comprises a piston tip seal (102) with a concave structure introduced at the end of the said modular piston (103) with a movable structure.

The invention relates to an injector arrangement (100) which allows the fat tissue to be drawn through a suction tip (108), stored inside a tube (101) without intervention or exposure to air and centrifuged finally in order to obtain fat based stem cells. It comprises a modular piston (103) with a movable structure which allows drawing the fat tissue into the tube (101) through the said suction tip (108) and a piston shaft (105) with modular structure which allows the said modular piston (103) to move inside the tube (101) in forward and backward directions and which can be separated from the modular piston (103).

It comprises threaded tips (106) which allow the said modular piston shaft (105) to be separated from the modular piston (103) and a modular piston screw hole (1032) introduced on the upper part of the modular piston (103) which allows the said threaded tips (106) to be removed and fastened. In addition, the said modular piston shaft (105) comprises a piston handle (107). On the other hand, the said modular piston (103) comprises at least one sealing O-ring (104). It also comprises a piston tip seal (102) comprising a positioning gap (1021) which is confined by the said modular piston (103) inside the tube (101) and in which the modular piston (103) is centered. Furthermore, it comprises a separate hollow locking part (1031) with a diameter larger than the inner tube (101) diameter of the said modular piston (103).

The operation of the injector arrangement (100) is as follows; when the modular piston (103) is pulled towards the a-direction, stem cells are sucked or drawn into the tube (101) through the suction tip (108). When the modular piston (103) is pulled towards the a-direction following a linear motion, the piston tip seal (102) advances inside the tube (101) with a leak tight contact and generates high suction power. Once the stem cells are confined inside the tube (101), the piston shaft (105) moves freely inside the piston shaft seat (109) which is formed vertically at the axial center of the hollow locking part (1031). The modular piston shaft (105) passes through the piston shaft seat (109) and unmounted from the modular piston screw hole (1032) by means of the threaded tips (106). The modular piston shaft (105) and the modular piston (103) which are engaged by being screwed are thereby separated from each other by twisting. Once stem cells are drawn into the tube (101), the piston shaft (105) is separated from the modular piston (103) by twisting. Thereby, the piston tip seal (102) and the hollow locking part (1031) of the modular piston (103) is maintained fastened inside the tube (101). Thereafter the stem cells inside the tube (101) are isolated from the air by means of a plug and a leak tight lid at the suction tip (108). In order to ensure a strong seal inside the tube (101) and prevent the stem cells which are being drawn into the tube (101) during the centrifuge to be thrown out of the tube (101) due to the centrifugal force, sealing O-rings (104) are placed inside the O-ring seat (1033) introduced on the hollow locking part (1031). The said sealing O-ring (104) ensures the hollow locking part (1031) to be held fixed against the centrifugal force during the centrifuge, in addition to ensuring tightness.

The said modular piston shaft (105) engages to the modular piston (103) by means of a threaded connection. This screw connection is established by engaging the modular piston screw hole (1032) introduced on the modular piston (103) and the threaded tip (106) introduced on the piston shaft (105) by twisting. Once the stem cells are drawn into the tube (101), the said piston shaft is removed by twisting before commencing the centrifuge process. Thereby, the length of the injector (100) is reduced in order to fit into the centrifuge. On the other hand, depending on the choice of the connection type, it is possible to establish the fastening by means of tooth, snap or any other connection technique.

In the injector arrangement developed by the present invention, a piston tip seal (102) having a concave structure is introduced at the tip of the said modular piston (103) with movable structure. The concavity of the said piston tip seal (102) prevents the stem cells to leak in between the seal and the outer wall of the injector during the centrifuge process in order to prevent volume losses.

## Claims

1. An injector arrangement (100) which allows the fat tissue to be drawn through a suction tip (108), stored inside a tube (101) without intervention or exposure to air and centrifuged finally in order to obtain fat based stem cells, comprising a modular piston (103) with a movable structure which allows drawing the fat tissue into the tube (101) through the said suction tip (108) by means of a piston tip seal (102), a piston shaft (105) with modular structure which allows the said modular piston (103) to move inside the tube (101) in forward and backward directions and which can be separated from the modular piston (103) in order to allow the tube (101) to be placed inside a centrifuge device after the fat tissue is drawn inside the tube (101), and an O-ring (104) which engages into the O-ring seat (1033) formed on a hollow locking part (1031) with independent structure in order to make the hollow locking part (1031) with an independent structure introduced on the said modular piston (103) to act as a locking device during the centrifuge process, such as to prevent the fat tissue and split stem cells to leak outside the tube (101) during the centrifuge, wherein; the injector arrangement (100) comprises said suction tip (108), said tube (101), said piston tip seal (102) and said hollow locking part (1031) and wherein said piston tip seal (102) has a concave structure.

## Patentansprüche

1. Injektoranordnung (100), die es ermöglicht, das Fettgewebe durch eine Saugspitze (108) anzusaugen, ohne Eingriff oder Aussetzen der Luft in einem Rohr (101) aufzubewahren und schließlich zu zentrifugieren, um Stammzellen auf Fettbasis zu erlangen, umfassend einen modularen Kolben (103) mit einer beweglichen Struktur, die es ermöglicht, das Fettgewebe mittels einer Kolbenspitzendichtung (102) durch die Saugspitze (108) in das Rohr (101) anzusaugen, einen Kolbenschaft (105) mit modularem Aufbau, der es ermöglicht, dass sich der modulare Kolben (103) innerhalb des Rohrs (101) in Vorwärts- und Rückwärtsrichtung bewegt, und der von dem modularen Kolben (103) getrennt werden kann, um zu ermöglichen, dass das Rohr (101) in einer Zentrifugenvorrichtung angeordnet wird, nachdem das Fettgewebe in das Rohr (101) angesaugt wurde, und einen O-Ring (104), der in den O-Ring-Sitz (1033) eingreift, der an einem hohlen Verriegelungsteil (1031) mit unabhängiger Struktur gebildet ist, um zu bewirken, dass das hohle Verriegelungsteil (1031) mit einer unabhängigen Struktur, das in den modularen Kolben (103) eingeführt ist, während des Zentrifugiervorgangs als eine Verriegelungsvorrichtung wirkt, um zu verhindern, dass das Fettgewebe und die aufgespaltenen Stammzellen während des Zentrifugierens aus dem Rohr (101) austreten, wobei; die Injektoranordnung (100) die Saugspitze (108), das Rohr (101), die Kolbenspitzendichtung (102) und das hohle Verriegelungsteil (1031) umfasst und wobei die Kolbenspitzendichtung (102) eine konkave Struktur aufweist.

## Revendications

1. Agencement d'injecteur (100) qui permet l'aspiration du tissu graisseux par l'intermédiaire d'une pointe d'aspiration (108), rangée à l'intérieur d'un tube (101) sans intervention ni exposition à l'air et sa centrifugation finale afin d'obtenir des cellules souches à base de graisse, comprenant un piston modulaire (103) doté d'une structure mobile qui permet d'aspirer le tissu graisseux dans le tube (101) par l'intermédiaire de ladite pointe d'aspiration (108) au moyen d'un joint de pointe de piston (102), une tige de piston (105) dotée d'une structure modulaire qui permet le déplacement dudit piston modulaire (103) à l'intérieur du tube (101) dans les directions avant et arrière et qui peut être séparé du piston modulaire (103) afin de permettre la mise en place du tube (101) à l'intérieur d'un dispositif de centrifugation après l'aspiration du tissu graisseux à l'intérieur du tube (101), et un joint torique (104) qui s'engage dans le siège de joint torique (1033) formé sur une partie de verrouillage creuse (1031) dotée d'une structure indépendante afin de faire en sorte que la pièce de verrouillage creuse (1031) dotée d'une structure indépendante introduite sur ledit piston modulaire (103) agisse comme un dispositif de verrouillage pendant le processus de centrifugation, de manière à empêcher le tissu graisseux et les cellules souches fractionnées de fuir à l'extérieur du tube (101) pendant la centrifugation ; ledit agencement d'injecteur (100) comprenant ladite pointe d'aspiration (108), ledit tube (101), ledit joint de pointe de piston (102) et ladite pièce de verrouillage creuse (1031) et ledit joint de pointe de piston (102) présentant une structure concave.
